# EUROPEAN PATENT SPECIFICATION

(11) **EP 1 336 416 B1**
(45) Date of publication and mention of the grant of the patent: **09.03.2005**
(21) Application number: 03011119.9
(22) Date of filing: 30.11.2001
(51) Int. Cl.: A61L 31/04

(54) **Cuff**
Manchette
Manschette

(30) Priority: 30.11.2000 JP 2000364424
(43) Date of publication of application: 20.08.2003
(62) Divisional of application: 01128358.7
(73) Proprietor: Kuraray Co., Ltd., Kurashiki-shi, Okayama-ken (JP)
(72) Inventor: Ogushi, Masayasu, Kuraray Co., Ltd., Ibaraki-ken (JP); Fukuda, Motohiro, Kuraray Co., Ltd., Ibaraki-ken (JP); Zento, Toshiyuki, Kuraray Co., Ltd., Ibaraki-ken (JP); Kirita, Yasuzo, Kuraray Co., Ltd., Osaka-shi, Osaka-fu (JP); Nakashima, Toshihide, Kuraray Co., Ltd., Okayama-ken (JP); Fujieda, Yukihiro, Kuraray Co., Ltd., Okayama-ken (JP)
(74) Representative: HOFFMANN - EITLE

(56) References cited:
- GB-A- 2 108 971
- DATABASE WPI Section Ch, Week 199820 Derwent Publications Ltd., London, GB; Class A18, AN 1998-225233 XP002194181 & JP 10 067894 A (KURARAY CO LTD) 10 March 1998 (1998-03-10)

## Description

### BACKGROUND OF THE INVENTION

### Field of the Invention

The present invention relates to a cuff for an endotracheal tube. More specifically, the present invention relates to a cuff for such a tube such as for an orally inserted endotracheal tube, a nasally inserted endotracheal tube, and a tube for tracheostomy to be inserted into the trachea from a tracheostoma.

### Discussion of the Related Art

An endotracheal tube is a medical tool used for an anesthetic treatment during surgery. A plasticized-polyvinyl chloride has been used in many of endotracheal tubes in consideration of mechanical strength, transparency and costs, as well as giving appropriate flexibility. However, the plasticized-polyvinyl chloride may generate a harmful substance such as dioxin when the plasticized-polyvinyl chloride is burned. Also, since a plasticizer such as dioctyl phthalate incorporated into the plasticized polyvinyl chloride is regarded as a harmful substance in the environment, the plasticized-polyvinyl chloride is not preferable for medical tools.

GB-A-2 108 971 discloses elastomeric compositions for making endotracheal tube cuffs comprising a mixture of an elastomeric hydrocarbon block copolymer and 0,1-12 percent by weight of a polysiloxane. The block copolymer may comprise blocks of Styerene-ethylene-butadiene-styrene. Mineral oil and polypropylene are preferably dispersed in the composition.

### SUMMARY OF THE INVENTION

According to the present invention, there is provided a cuff for an endotracheal tube having a storage modulus of not more than 5.0 x 10⁷ N/m² at 25°C obtainable by subjecting a resin composition comprising a styrenic elastomer and a polyolefin in a weight ratio within the range 60/40 to 80/20 to blow-molding, wherein the resin composition has a melt tension of not less than 1 g at 230°C.

Preferably the weight ratio of the styrenic elastomer to the polyolefin is 70/30 to 80/20.

### BRIEF DESCRIPTION OF THE DRAWINGS

Figure 1 is a schematic explanatory view showing one embodiment of an endotracheal tube comprising a cuff of the present invention;
Figure 2 is a schematic explanatory view an extrusion blow molding machine used in, each Example and each Comparative Example; and
Figure 3 is a schematic explanatory view of a cuff obtained in each of Examples 1 to 4 and Comparative Examples 1 and 2.

### DETAILED DESCRIPTION OF THE INVENTION

In the endotracheal tube, a tube obtained by subjecting a resin composition comprising a styrenic elastomer and a polyolefin to extrusion-molding is used as a main tube.

As the polyolefin, various polyolefins made of olefin monomers can be used. The polyolefin includes, for instance, polyethylenes such as high-density polyethylene, low-density polyethylene, linear low-density polyethylene and high pressure processed ethylene-α-olefin copolymer; polypropylenes such as propylene homopolymer; a random copolymer of ethylene and propylene; a block-type polypropylene comprising ethylene blocks; terpolymers of propylene, ethylene and butene-1; and the like. Each of those polyolefins can be used alone or in admixture of at least two kinds. Among those polyolefins, the polypropylenes are especially preferable.

As to the melt viscosity of the polyolefm, the melt flow rate (MFR) of the polyolefin is within the range of preferably 0.1 to 500, more preferably 2 to 200, as determined by the method in accordance with ASTM D-1238 at 230°C under the load of 2160 g.

It is preferable that the styrenic elastomer is a block copolymer of a styrenic polymer block (A) and a hydrogenated conjugated diene polymer block (B).

The styrenic polymer block (A) is made of a styrenic monomer. Examples of the styrenic monomer are, for instance, styrene, α-methylstyrene, 3-methylstyrene, 4-propylstyrene, 4-cyclohexylstyrene, 4-dodecylstyrene, 2-ethyl-4-benzylstyrene, 4-(phenylbutyl)styrene, and the like. Those monomers can be used alone or in admixture of at least two kinds. Among those styrene monomers, styrene is preferable.

The number-average molecular weight of the styrenic polymer block (A) is not limited to specified ones. It is preferable that the number-average molecular weight is within the range of 2500 to 20000.

The content of the styrenic polymer block (A) in the block copolymer is preferably not less than 10% by weight, more preferably not less than 15% by weight, from the viewpoint of improving the mechanical strength of the block copolymer. Also, the content of the styrenic polymer block (A) in the block copolymer is preferably not more than 40% by weight, more preferably not more than 30% by weight, from the viewpoint of facilitating homogeneous mixing with the polyolefin. Accordingly, from these viewpoints, the content of the styrenic polymer block (A) in the block copolymer is preferably 10 to 40% by weight, more preferably 15 to 30 % by weight.

It is preferable that the hydrogenated conjugated diene polymer block (B) has at least one polymer block selected from the group consisting of a hydrogenated polyisoprene block (B1), a hydrogenated isoprene/butadiene copolymer block (B2) and a hydrogenated polybutadiene block (B3), from the viewpoint of the balance of flexibility and economics.

It is preferable that the hydrogenated polyisoprene block (B1) is a hydrogenated polyisoprene block made of a polyisoprene having a 1,2-bond and 3,4-bond content (hereinafter simply referred to as "content of vinyl bonds") of 10 to 75% by mol, in which not less than 70% of carbon-carbon double bonds of the polyisoprene are hydrogenated.

The content of vinyl bonds in the hydrogenated polyisoprene block (B1) is preferably not less than 10% by mol, more preferably not less than 20% by mol, from the viewpoint of increasing the transparency of an endotracheal tube. Also, the content of vinyl bonds is preferably not more than 75% by mol, more preferably not more than 65% by mol, from the viewpoint of not making a glass transition temperature (Tg) of the hydrogenated polyisoprene block (B1) exceedingly high, thereby giving the endotracheal tube appropriate flexibilities. Accordingly, from these viewpoints, the content of vinyl bonds in the hydrogenated polyisoprene block (B1) is preferably 10 to 75% by weight, more preferably 20 to 65% by weight.

In addition, the ratio of hydrogenation of carbon-carbon double bonds of the polyisoprene is preferably not less than 70%, more preferably not less than 80% by mol, from the viewpoints of increasing its compatibility with the polyolefin, thereby giving the endotracheal tube excellent transparency.

The number-average molecular weight of the hydrogenated polyisoprene block (B1) is not limited to specified ones. It is preferable that the number-average molecular weight is within the range of 10000 to 200000.

It is preferable that the hydrogenated isoprene/butadiene copolymer block (B2) is a hydrogenated isoprene/butadiene copolymer block comprising an isoprene/butadiene copolymer having a 1,2-bond and 3,4-bond content, i.e. the content of vinyl bonds, of 20 to 85% by mol, obtained by copolymerizing isoprene and butadiene in a weight ratio of 5/95 to 95/5, wherein not less than 70% of carbon-carbon double bonds are hydrogenated

The weight ratio of isoprene/butadiene is preferably not more than 95/5, more preferably not more than 80/20, from the viewpoints of not making the glass transition temperature (Tg) exceedingly high when the content of vinyl bonds in the hydrogenated isoprene/butadiene copolymer block (B2) is not less than 75% by mol, and increasing the flexibility of the endotracheal tube. Also, the weight ratio of isoprene/butadiene is preferably not less than 5/95, more preferably not less than 20/80, from the viewpoint of increasing the transparency of the endotracheal tube when the content of vinyl bonds in the hydrogenated isoprene/butadiene copolymer block (B2) is less than 30% by mol. Accordingly, from these viewpoints, the weight ratio of isoprene/butadiene is preferably 5/95 to 95/5, more preferably 20/80 to 80/20.

It is desired that carbon-carbon double bonds of the isoprene/butadiene copolymer are hydrogenated in a ratio of not less than 70%, preferably not less than 80%, from the viewpoints of increasing its compatibility with the polyolefin and improving the transparency of the endotracheal tube.

The content of vinyl bonds in the hydrogenated polyisoprene/butadiene copolymer block is preferably not less than 20% by mol, more preferably not less than 40% by mol, from the viewpoint of increasing the transparency of the endotracheal tube. Also, the content of vinyl bonds is preferably not more than 85% by mol, more preferably not more than 70% by mol, from the viewpoints of not making a glass transition temperature (Tg) of the hydrogenated polyisoprene block (B1) exceedingly high, thereby giving the endotracheal tube appropriate flexibilities. Accordingly, from these viewpoints, the content of vinyl bonds in the hydrogenated polyisoprene/butadiene copolymer block is preferably 20 to 85% by weight, more preferably 40 to 70% by weight.

The polymerization form of isoprene and butadiene in the hydrogenated isoprene/butadiene copolymer block (B2) is not limited to specified ones, and can be any of random, block and tapered forms.

The number-average molecular weight of the hydrogenated isoprene/butadiene copolymer block (B2) is not limited to specified ones. It is preferable that the number-average molecular weight is within the range of 10000 to 200000.

It is preferable that the hydrogenated polybutadiene block (B3) is a hydrogenated polybutadiene block made of a polybutadiene having a 1,2-bond and 3,4-bond content, i.e. the content of vinyl bonds, of not less than 45% by mol, wherein not less than 70% of carbon-carbon double bonds of the polybutadiene are hydrogenated.

The content of vinyl bonds in the polybutadiene is preferably not less than 45% by mol, more preferably 60 to 80% by mol, from the viewpoint of increasing the transparency of the endotracheal tube.

The ratio of hydrogenation of carbon-carbon double bonds of the polybutadiene is preferably not less than 70%, more preferably not less than 80% by mol, from the viewpoints of increasing its compatibility with the polyolefin, thereby increasing transparency of the endotracheal tube.

The number-average molecular weight of the hydrogenated polybutadiene block (B3) is not limited to specified ones. The number-average molecular weight is preferably within the range of 10000 to 200000.

The content of the hydrogenated conjugated polymer block (B) in the block copolymer is preferably not more than 90% by weight, more preferably not more than 85% by weight, from the viewpoint of improving mechanical strength of the block copolymer, and the content is preferably not less than 60% by weight, more preferably not less than 70% by weight, from the viewpoint of facilitating homogeneous mixing of the block copolymer with the polyolefin. Accordingly, from these viewpoints, the content of the hydrogenated conjugated diene polymer block (B) in the block copolymer is preferably 60 to 90% by weight, more preferably 70 to 85% by weight.

In the block copolymer of the styrenic polymer block (A) and the hydrogenated conjugated diene polymer (B), the bonding forms of the styrenic polymer block (A) and the hydrogenated conjugated diene polymer block (B) are not limited to specified ones, and can be any of linear form, branched form and a given combination of these.

When the styrenic polymer block (A) is represented by "A", and the hydrogenated conjugated diene polymer block (B) is represented by "B", the molecular structure of the block copolymer includes the forms such as A-(B-A)ₙ and (A-B)ₙ, wherein n is an integer of not less than 1. The molecular structure of the block copolymer before hydrogenation may have a star-like form together with a coupling agent such as divinylbenzene, a tin compound or a silane compound, such as (A-B)ₘX, wherein m is an integer of not less than 2, and X is a residue of a coupling agent.

As the block copolymer, those having the above-mentioned molecular structures can be used alone, or in admixture of at least two different molecular structures, such as a mixture of triblock-type and diblock-type.

The number-average molecular weight of the block copolymer is not limited to specified ones. The number-average molecular weight is preferably within the range of 30000 to 300000.

The endotracheal tube comprises a tube obtained by subjecting the resin composition comprising a styrenic elastomer and a polyolefin to extrusion-molding.

The tube has a storage modulus of 5.0 × 10⁶ - 8.0 × 10⁷ N/m² (5.0 × 10⁷ to 8.0 × 10⁸ dyne/cm²) in the extrusion direction (MD) at 25°C, and a ratio of the storage modulus in the extrusion direction (MD) to a storage modulus in the circumferential direction (TD), i.e. MD/TD, of not more than 1.3 at 25°C.

The storage modulus can be determined by using a general dynamic viscoelasticity analyzer, for instance, Rheospectra commercially available from Rheology under the trade mane of DVE-V4 FT Rheospectra and the like.

The tube has a storage modulus in the extrusion direction (MD) of not less than 5.0 × 10⁶ N/m² (5.0 × 10⁷ dyne/cm²), preferably not less than 7.0 × 10⁶ N/m² (7.0 × 10⁷ dyne/cm²), more preferably not less than 8.0 × 10⁶ N/m² (8.0 × 10⁷ dyne/cm²) at 25°C, from the viewpoint of preventing the tube from being too flexible, thereby facilitating insertion of the tube into the trachea. Also, the tube has a storage modulus in the extrusion direction (MD) of not more than 8.0 × 10.⁷ N/m² (8.0 × 10⁸ dyne/cm²), preferably not more than 4.0 × 10⁷ N/m² (4.0 × 10⁸ dyne/cm²), more preferably not more than 2.0 × 10⁷ N/m² (2.0 × 10⁸ dyne/cm²) at 25°C, from the viewpoint of preventing the tube from becoming too rigid, thereby avoiding the generation of damages in the trachea. Accordingly, from the viewpoints, the tube has a storage modulus in the extrusion direction (MD) of 5.0 × 10⁶ - 8.0 × 10⁷ N/m² (5.0 × 10⁷ to 8.0 × 10⁸ dyne/cm²), preferably 7.0 × 10⁶ - 4.0 × 10⁷ N/m² (7.0 × 10⁷ to 4.0 × 10⁸ dyne/cm²), more preferably 8.0 × 10⁶ - 2.0 × 10⁷ N/m² (8.0 × 10⁷ to 2.0 × 10⁸ dyne/cm²).

The cuff provided by the present invention is adapted for use on the outer peripheral surface of such an endothracheal tube.

The cuff is made of a resin composition comprising a styrenic elastomer and a polyolefin.

As the polyolefin, various polyolefins made of olefinic monomers can be used. The polyolefin includes, for instance, polyethylenes such as high-density polyethylene, low-density polyethylene, linear low-density polyethylene and high pressure processed ethylene-α-olefin copolymer; polypropylenes such as propylene homopolymer; a random copolymer of ethylene and propylene; a block-type polypropylene containing ethylene blocks; terpolymers of propylene, ethylene and butene-1; and the like. These polyolefins can be used alone or in admixture of at least two kinds. Among those polyolefins, a polyolefin in which a cross-linking structure is introduced by applying electron beam irradiation is preferable in order to increase the melt tension during blow-molding,.

As the styrenic elastomer, the same ones as those styrenic elastomers used in the above-mentioned main tube can be exemplified. The styrenic elastomer is preferably a block copolymer suitably used for the above-mentioned tube. In addition, there can be used cross-linked block copolymers prepared by applying electron beam irradiation to the block copolymer, or a cross-linking the block copolymer with a radical such as a peroxide, in order to increase the melt tension during blow-molding.

The resin composition constituting the cuff has a melt tension of not less than 1 g, preferably not less than 1.5 g at 230°C, from the viewpoints of avoidance of the generation of the draw-down of the parison and breakage of the cuff at the draw-up during blow-molding. The melt tension of the resin composition at 230°C is determined by the method described below.

The cuff is produced by blow-molding the above-mentioned resin composition. When the storage modulus of the cuff at 25°C is too high, the cuff becomes too rigid, so that the cuff which has been shrunk under a pressure of 25 cm H₂O for expanding in a usual trachea would not be sufficiently expanded, and thereby the sealability of the endotracheal tube would be lowered. When the cuff is expanded until the sealing becomes sufficient, the blood capillary in the trachea is pressurized by the internal pressure of the cuff, so that the necrosis of the tissue tends to occur. Therefore, in the present invention, in consideration of these matters, the storage modulus of the cuff is controlled to not more than 5.0 × 10⁷ N/m² (5.0 × 10⁸ dyne/cm²) at 25°C.

The storage modulus can be determined by using the above-mentioned dynamic viscoelasticity analyzer.

The melt tension of the above-mentioned resin composition at 230°C and the storage modulus of the cuff molded therefrom at 25°C can be controlled by adjusting the ratio of the styrenic elastomer to the polyolefin.

In order to satisfy the above-mentioned melt tension of the resin composition at 230°C and the above-mentioned storage modulus of the cuff molded wherefrom at 25°C, the weight ratio of the styrenic elastomer to the polyolefin (styrenic elastomer/polyolefin) is within the range of 60/40 to 80/20, preferably 70/30 to 80/20.

It is preferable that the resin composition constituting the cuff contains at least one member selected from inorganic fillers and organic cross-linked particles, in order to prevent the cuff from uneven expansion due to sticking during the expansion of the cuff.

The inorganic filler includes, for instance, talc, calcium carbonate, mica, and the like. The organic cross-linked particles include, for instance, cross-linked acrylic resin beads, cross-linked polyurethane beads, cross-linked polystyrene beads and the like. Those inorganic fillers and organic cross-linked particles can be used alone or in admixture of at least two kinds.

The content of at least one member selected from inorganic fillers and organic cross-linked particles in the resin composition constituting the cuff is preferably not less than 5% by weight, from the viewpoint of sufficiently exhibiting the prevention of sticking. Also, the content is preferably not more than 20% by weight, more preferably not more than 10% by weight, from the viewpoint of improving the surface property of the cuff. Accordingly, from these viewpoints, the content is with the range of preferably 5 to 20% by weight, more preferably 5 to 10% by weight.

In the resin composition constituting the above-mentioned tube and cuff, there can be added various additives, including, for instance, an antioxidant, an ultraviolet absorbing agent, a light stabilizer, a colorant, a crystalline nucleus-forming agent, within a range which would not impair the properties of the resin composition. The amount of those additives cannot be absolutely determined because the amount differs depending upon their kinds. It is preferable that the amount of the additive is usually 0.01 to 5 parts by weight based on 100 parts by weight of the resin compositions.

To the above-mentioned resin composition, there can be added a softening agent such as a mineral oil, within the range which would not impair the properties of the resin composition. It is preferable that the amount of the softening agent is usually not more than 100 parts by weight based on 100 parts by weight of the resin composition.

Furthermore, there can be added to the above-mentioned resin composition other polymers, including, for instance, hydrogenated polyisoprene, hydrogenated polybutadiene, hydrogenated styrene-butadiene random copolymer, hydrogenated styrene-isoprene random copolymer, ethylene-vinyl acetate copolymer, ethylene-methacrylic acid copolymer, ethylene-acrylic acid copolymer, and their ionomers, ethylene-methyl acrylate copolymer and the like within the range which would not impair the properties of the resin composition.

The endotracheal tube can be produced by subjecting the above-mentioned resin composition to extrusion-molding to give a main tube, cutting the main tube to have an appropriate inner diameter satisfying the size prescribed in ISO 5361/1 (Tracheal Tube-Part 1: General Requirement), cutting slantwise one end of the tube, which is to be inserted into a patient at a bevel angle of 38°±10°, rounding off the cut edges by heat treatment, and subjecting the tube to a curving treatment.

When an endotracheal tube having a cuff is produced by extrusion molding, generally, the main tube is provided with a sublumen for passing air through the tube, which is used for expanding a cuff. Thereafter, the cuff is molded into a spindle-like shape or Rugby foot ball-like shape, and bonded to the outer periphery of the main tube at the portion to be inserted to a patient.

It is usually preferable that the main tube is previously provided with a notch communicating with the sublumen for expanding the cuff at the portion to be bonded with the cuff. The endotracheal tube having a cuff is obtained by bonding one end of a tail tube to the sublumen within the size range as defined in ISO 5361/1, and bonding the other end of the tail tube to a pilot balloon, a check valve and the like.

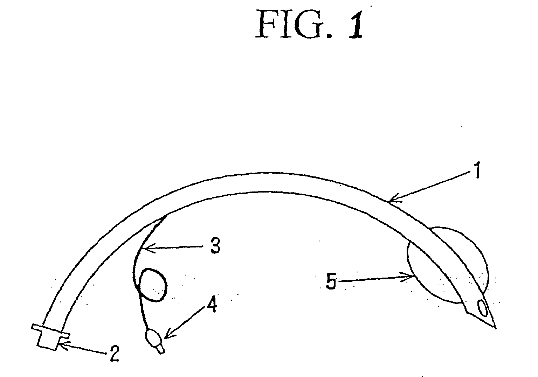

An embodiment of the endotracheal tube produced by the above-mentioned method is shown in Figure 1.

Figure 1 is a schematic explanatory view showing one embodiment of an endotracheal tube in which a connector 2 is provided on one end of a main tube 1 having a cuff 5. In Figure 1, one end of a tail tube 3 is bonded to a sublumen (not shown) of the main tube 1, and the other end of the tail tube 3 is provided with a pilot balloon 4.

### EXAMPLES

Next, the present invention will be described more specifically on the basis of the examples, without intending to limit the present invention thereto.

The resins, the molding machine, and the determination methods used in Examples and Comparative Examples are as follows.

| [Resin] | |
|---|---|
| Resin 1 | Polypropylene (random-type, commercially available from Grand Polymer under the trade name of F327) |
| Resin 2 | Polypropylene (homo-type, commercially available from Montel under the trade name of SD613) |
| Resin 3 | Polyethylene (low-density polyethylene, commercially available from Nippon Polychem K.K. under the trade name of HE30) |
| Resin 4 | Styrenic elastomer [hydrogenated SIS (hydrogenated styrene-isoprene-styrene block copolymer), commercially available from Kuraray Co., Ltd. under the trade name of HYBRA HVS7125, number-average molecular weight 100000, styrene content: 20% by weight, hydrogenation ratio: 90%, content of vinyl bond: 55% by mol] |

### [Extruder]

φ40 mm single-screw extruder (commercially available from Osaka Seiki)

### [Molding Machine]

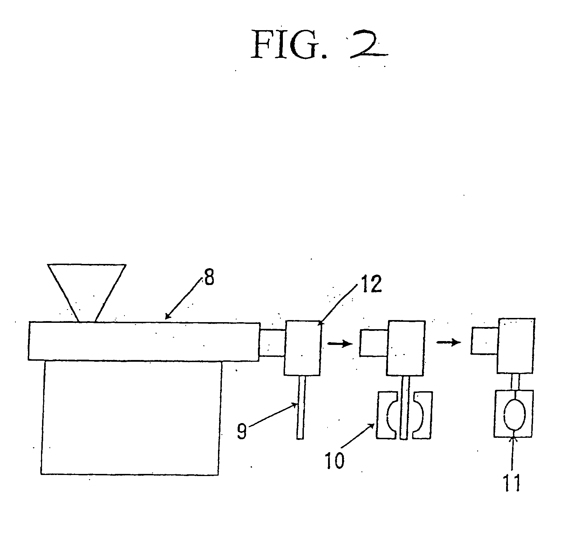

An extrusion blow molding machine: a φ22 mm single-screw extruder as shown in Figure 2 was used (commercially available from Pla-eng). Figure 2 is a schematic view of the extrusion blow molding machine. The φ22 mm single-screw extruder was provided with a die 12, and a parison 9 made of a thermally melted resin composition was extruded from the die 12. Next, the resulting parison 9 was inserted between two split dies having a desired internal shape, and the split dies were clamped together. Thereafter, air was blown into the die 12 from an air mandrel (not shown) of the die 12, thereby giving a blow-molding product having a given shape. In Figure 2, 11 is an air blow provided at the bottom of the split dies 10.

### [Determination of Physical Properties]

### 1. Storage Modulus

The storage modulus was determined by tensile-type dynamic viscoelasticity device commercially available from Rheology under the trade name of DVE-V4 FT Rheospectra (determination temperature: 25°C, shape of cross section of the sample: 1 mm in thickness and 5 mm in width, distance between chucks: 10 mm, strain ratio: 0.03%, frequency: 1 Hz/sine wave, static load: automatic static load control).

### 2. Melt Tension

The melt tension was determined by using a capillograph (Shimadzu Corporation), a melt tension determination device in accordance with the following method.

A resin composition was preheated at 230°C for 4 minutes in a cylinder, and thereafter discharged from a capillary (φ1 mm, L/D = 10) with a piston at a rate of 20 mm/min. A strand was taken off at a given speed of 10 m/min, and a load was read off and recorded at a stress gauge via a pulley positioned in the course of take-off to start taking determinations. The melt tension was defined as an average value of the load reading at a point of 20 seconds after the load curve was stabilized.

### Reference Example 1

A resin composition was prepared by mixing the polypropylene (Resin 1) and the styrenic elastomer (Resin 4) in a weight ratio of 30/70, and stearic acid monoglyceride or oleic amide was added to the resin composition in a proportion as shown in Table 1. The mixture was molded with an extruder (φ40 mm) at a resin temperature of 200°C, to give an endotracheal tube having an inner diameter of φ7 mm and an outer diameter of φ11 mm. The operability when a suction catheter made of a plasticized-polyvinyl chloride was inserted into the internal of the resulting tube and the printability on the outer surface of the tube were evaluated. The results are shown in Table 1.

**Table 1**

| | Resin Composition (% by wt.) | Stearic Acid Monoglyceride (% by wt.) | Oleic Amide (% by wt.) | Operability | Printability |
|---|---|---|---|---|---|
| Ref. Ex. 1 | 99.95 | 0.05 | - | Excellent | ○ |
| Note: 1) Resin Composition: Resin 1/Resin 4 = 30/70 (weight ratio) | | | | | |
| 2) Operability: The operability was evaluated by inserting a suction catheter into a tube of each example to detect the resistance felt by touch. | | | | | |
| 3) Printability: An ink for polypropylene was applied to a tube surface, and dried, and thereafter the surface was subjected to scotch tape peeling test. ○: no peeling, ×: peeling | | | | | |

### Example 1

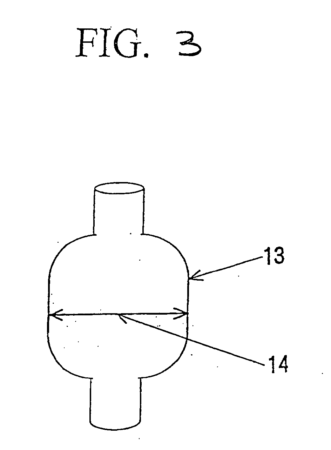

A resin composition was prepared by mixing the polypropylene (Resin 2) and the styrenic elastomer (Resin 4) in a weight ratio of 25/75, and molded by using the extrusion blow molding machine as shown in Figure 2 to give a spindle-like cuff 13 having the shape as shown in Figure 3. The resulting spindle-like cuff 13 had a cuff diameter 14 of about 30 mm and a thickness of 30 to 100 µm.

As to the resulting spindle-like cuff 13, melt tension of the resin composition, blow moldability during molding, and storage modulus are shown in Table 2.

Next, an endotracheal tube was produced by using this cuff and the tube obtained in Reference Example 1. The endotracheal tube was inserted into a trachea of a pig, and the endotracheal sealability was evaluated when the cuff was expanded at a pressure of 25 cm H₂O. The results are also shown in Table 2.

### Example 2

A cuff and an endotracheal tube were produced in the same manner as in Example 1 except that a resin composition was prepared by mixing a polypropylene (Resin 2), a polyethylene (Resin 3) and a styrenic elastomer (Resin 4) in a weight ratio of 12.5/12.5/75. The physical properties of the resulting cuff and the endotracheal tube were evaluated in the same manner as in Example 1. The results are shown in Table 2.

### Comparative Example 1

A cuff and an endotracheal tube were produced in the same manner as in Example 1 except that a resin composition was prepared by mixing a polypropylene (Resin 2) with a styrenic elastomer (Resin 4) in a weight ratio of 50/50. The physical properties of the resulting cuff and the endotracheal tube were evaluated in the same manner as in Example 1. The results are shown in Table 2.

### Comparative Example 2

A cuff and an endotracheal tube were attempted to be produced in the same manner as in Example 1 except that a resin composition was prepared by mixing a polypropylene (Resin 1) with a styrenic elastomer (Resin 4) in a weight ratio of 25/75. However, the cuff was broken during blow-molding, so that a cuff having good physical properties could not be obtained. The results are shown in Table 2.

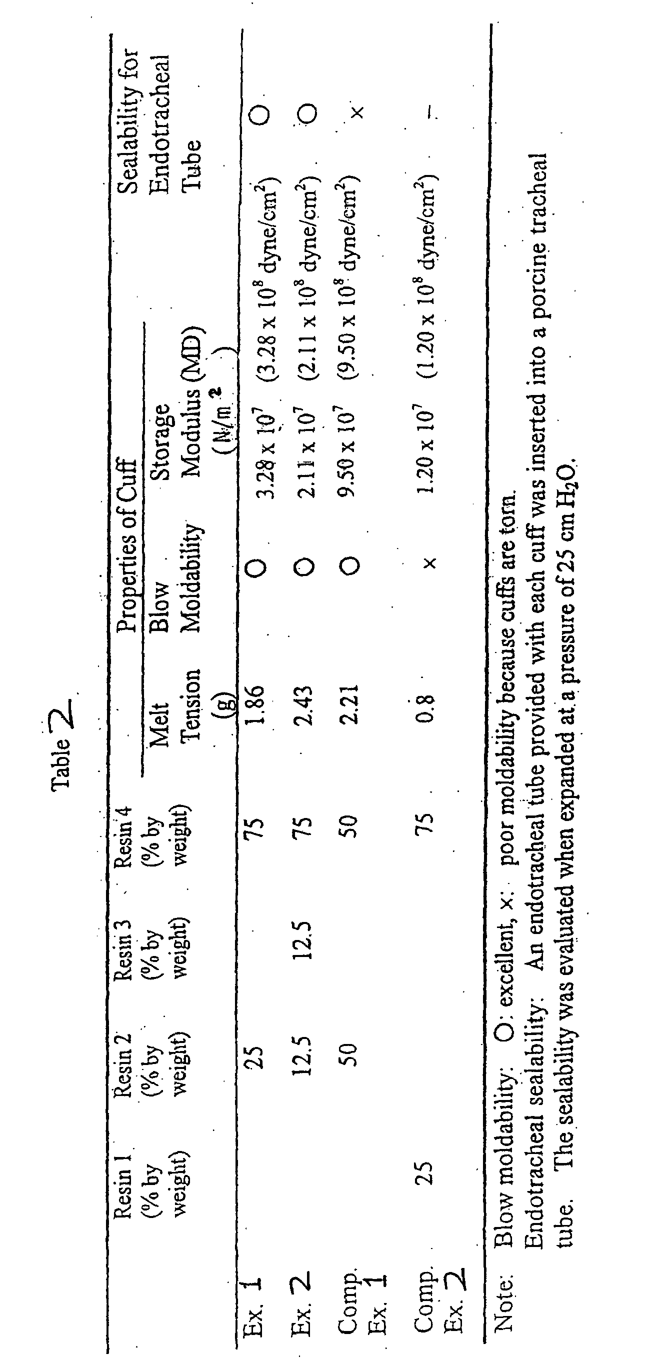

It can be seen from the results shown in Table 2 that the cuffs obtained in Examples 1 and 2 have storage moduli of not more than 5.0 × 10⁷ N/m² (5.0 × 10⁸ dyne/cm²), and melt tension of the resin composition is not less than 1 g, so that the cuffs are excellent in the blow moldability. Also, since the resin compositions are flexible, the resulting cuffs are excellent in its endotracheal sealability when the cuff is expanded at a pressure of 25 cm H₂O in the trachea.

On the other hand, it can be seen in Comparative Example 1 that since a resin composition having a storage modulus greater than 5.0 × 10⁷ N/m² (5.0 × 10⁸ dyne/cm²) is used, the resulting cuff is excellent in blow-moldability, but the cuff has a hard texture, so that the tube is insufficient in the endotracheal sealability during expansion in the trachea.

In addition, it can be seen in Comparative Example 2 that since a resin composition having a melt tension of less than 1 g is used, the cuff is broken during blow-molding, so that a cuff having excellent physical properties cannot be obtained.

### Examples 3 and 4

A cuff and an endotracheal tube were produced in the same manner as in Example 1 except that talc (the Japanese Pharmacopoeia) was added in a ratio as shown in Table 3 to a resin composition prepared by mixing a polypropylene (Resin 2) and a styrenic elastomer (Resin 4) in a weight ratio of 25/75.

Prevention of sticking between the cuff and the main tube of the resulting endotracheal tube, and the surface property of the cuff, i.e. external appearance, were evaluated. The results are shown in Table 3.

**Table 3**

| | Resin Composition (% by wt.) | Talc (% by wt.) | Prevention of Sticking | Surface Property |
|---|---|---|---|---|
| Ex. 3 | 95 | 5 | ○ | ○ |
| Ex. 4 | 80 | 20 | ○ | ○ |
| Note: 1) Resin Composition: Resin 2/Resin 4 = 25/75 (weight ratio) | | | | |
| 2) Prevention of Sticking: The prevention of sticking between the cuff and the main tube was evaluated as follows: ○: no sticking, ×: sticking | | | | |
| 3) Surface property: The surface property of the cuff was visually examined as follows. ○: excellent surface property, ×: poor surface property | | | | |

It can be seen from the results shown in Table 3 that since the resin compositions constituting the cuffs contain 5 to 20% by weight of talc used in the cuffs of the endotracheal tubes obtained in Example 3 and 4, the cuffs are excellent in prevention of sticking and surface property.

## Claims

1. A cuff for an endotracheal tube having a storage modulus of not more than 5.0 x 10⁷ N/m² at 25°C obtainable by subjecting a resin composition comprising a styrenic elastomer and a polyolefin in a weight ratio within the range 60/40 to 80/20 to blow-molding, wherein the resin composition has a melt tension of not less than 1 g at 230°C.

2. A cuff according to Claim 1, wherein the weight ratio of the styrenic elastomer to the polyolefin is 70/30 to 80/20.

## Patentansprüche

1. Manschette für eine Endotrachealröhre mit einem Speichermodul von nicht mehr als 5,0 × 10⁷ N/m² bei 25°C, erhältlich durch Blasformgebung einer Harzzusammensetzung, die ein Styrolelastomer und ein Polyolefin in einem Gewichtsverhältnis von 60:40 bis 80:20 umfasst, worin die Harzzusammensetzung eine Schmelzspannung von nicht mehr als 1 g bei 230°C besitzt.

2. Manschette gemäss Anspruch 1, worin das Gewichtsverhältnis von Styrolelastomer zu Polyolefin 70:30 bis 80:20 beträgt.

## Revendications

1. Ballonnet destiné à un tube endotrachéal présentant un module de conservation ne dépassant pas 5,0 × 10⁷ N/m² à 25 °C, pouvant être obtenu en soumettant une composition de résine comprenant un élastomère de styrène et une polyoléfine, dans un rapport en poids dans la plage de 60/40 à 80/20, à un moulage par soufflage, dans lequel la composition de résine présente une tension de fusion qui n'est pas inférieure à 1 g à 230 °C.

2. Ballonnet selon la revendication 1, dans lequel le rapport en poids de l'élastomère de styrène sur la polyoléfine est de 70/30 à 80/20.
